# EUROPEAN PATENT APPLICATION

(11) **EP 1 598 347 A1**
(43) Date of publication of application: **23.11.2005**
(21) Application number: 05008036.5
(22) Date of filing: 13.04.2005
(51) Int. Cl.: C07D 401/12, A61K 31/4439, A61P 1/04

(54) **Polymorphs of pantoprazole sodium salt and process for the preparation thereof**

(30) Priority: 23.04.2004 IT MI20040802
(71) Applicant: Dipharma S.p.A., 33036 Mereto di Tomba (Udine) (IT)
(72) Inventor: Allegrini, Pietro, 20097 San Donato Milanese (MI) (IT); Ventimiglia, Gianpiero, 20092 Cinisello Balsamo (MI) (IT); Sommei, Antonio, Attard BLZ 02 (MT); Castaldi, Graziano, 28072 Briona (NO) (IT)
(74) Representative: Minoja, Fabrizio

(57) **Abstract**

Novel crystalline forms of pantoprazole sodium salt solvate with ketone solvents, a process for the preparation thereof, the use of said forms for the purification of pantoprazol, pharmaceutical compositions therefrom and the use thereof in therapy.

## Description

### FIELD OF THE INVENTION

The present invention relates to novel crystalline forms of pantoprazole sodium salt, in particular crystalline forms approximately monohydrate and monosolvate with ketone solvents, a process for the preparation thereof, pharmaceutical compositions therefrom and the use thereof in therapy.

### TECHNOLOGICAL BACKGROUND

Pantoprazole sodium, i.e. 5-(difluoromethoxy)-2-[[(3,4-dimethoxy-2-pyridinyl)methyl]sulfinyl]-1H-benzimidazole sodium salt, is used as inhibitor of the gastric acid secretion and is useful in the prevention and treatment of ulcer. Pantoprazole sodium is known to exist in two hydrated forms: a first approximately monohydrate form is disclosed in EP 533,790, whereas the sesquihydrate form, disclosed in EP 166,287, contains about 1.5 moles of water per mole of active ingredient and is the one commercially available at present. US 6,545,024 discloses complexes containing 1 to 4 molecules of some active principles having a sulfoxide group, including pantoprazol, with 1-4 molecules of acetone. US 6,545,024, however, provides no useful information on the characterization of said solvate, for example the number of acetone molecules per pantoprazole sodium molecule present in the crystallized product is not specified.

### SUMMARY OF THE INVENTION

It has now been found that pantoprazole sodium, in addition to the known crystalline mono and sesquihydrate forms and to the crystalline solvate form with acetone, can exist also in other novel forms that are stable at room temperature.

A first object of the invention is therefore a novel approximately monohydrate crystalline form of pantoprazole sodium as monosolvate with ketone solvents. A further object of the invention is a process for the preparation of said novel crystalline form. The invention also relates to a pharmaceutical composition comprising a diluent and/or carrier together with said novel crystalline form as active ingredient. The invention further relates to the use of said novel crystalline form as an intermediate in a process for the purification of pantoprazole sodium mono or sesquihydrate.

### BRIEF DESCRIPTION OF THE FIGURE AND TABLES

The novel forms were characterised using the known XRPD (X-ray powder diffraction) and ¹H-NMR techniques. Furthermore, the water content in the tested compounds was measured by titration with the known Karl Fischer method. The X-ray diffraction spectra (XRPD) were recorded with an APD-2000 automated θ/θ diffractometer for powders and liquids manufactured by Ital-Structures, under the following operative conditions: CuKα radiation (λ= 1.5418 Å), scanning angular range 0.03° for a time of 1 sec. The ¹H-NNR spectra were recorded with a Varian Mercury 300 instrumentation, using DMSO-d₆ as the solvent. The IR spectra were recorded with a FT-IR System 2000 Perkin Elmer spectrophotometer.
Fig. 1A. XRPD of approximately monohydrate pantoprazole sodium monosolvate with methyl ethyl ketone.
Fig. 1B. IR Spectrum of approximately monohydrate pantoprazole sodium monosolvate with methyl ethyl ketone.
Fig. 2A. XRPD of approximately monohydrate pantoprazole sodium monosolvate with methyl isobutyl ketone.
Fig. 2B. IR Spectrum of approximately monohydrate pantoprazole sodium monosolvate with methyl isobutyl ketone.
Fig. 3A. XRPD of approximately monohydrate pantoprazole sodium monosolvate with diethyl ketone.
Fig. 3B. IR Spectrum of approximately monohydrate pantoprazole sodium monosolvate with diethyl ketone.

**Table 1.**

| 2θ Values, interspace distances and relative intensities of the diffraction peaks of approximately monohydrate pantoprazole sodium monosolvate with methyl ethyl ketone; wherein the more intense diffraction peaks are observed at 5.31, 13.53, 15.66, 16.08, 18.51, 26.43 and 26.76 in 2θ. | | |
|---|---|---|
| Diffraction angles (2θ, °) | Interspace distances (Å) | I/Imax (%) |
| 5.310 | 16.6292 | 100 |
| 10.620 | 8.3236 | 18 |
| 11.550 | 7.6554 | 6 |
| 12.240 | 7.2253 | 9 |
| 13.530 | 6.5392 | 27 |
| 15.660 | 5.6542 | 35 |
| 16.080 | 5.5075 | 19 |
| 16.680 | 5.3107 | 13 |
| 18.090 | 4.8998 | 15 |
| 18.510 | 4.7896 | 19 |
| 18.990 | 4.6696 | 5 |
| 19.650 | 4.5142 | 5 |
| 19.980 | 4.4404 | 5 |
| 21.360 | 4.1565 | 8 |
| 21.780 | 4.0773 | 11 |
| 22.440 | 3.9588 | 6 |
| 23.280 | 3.8179 | 13 |
| 23.700 | 3.7511 | 9 |
| 24.780 | 3.5900 | 9 |
| 25.740 | 3.4583 | 11 |
| 26.430 | 3.3695 | 48 |
| 26.760 | 3.3827 | 27 |
| 27.450 | 3.2466 | 5 |
| 29.010 | 3.0755 | 9 |
| 29.940 | 2.9820 | 7 |
| 33.300 | 2.6884 | 7 |
| 33.720 | 2.6559 | 8 |
| 34.440 | 2.6020 | 8 |
| 35.010 | 2.5609 | 6 |
| 36.120 | 2.4847 | 4 |
| 36.570 | 2.4552 | 5 |
| 38.130 | 2.3582 | 7 |

**Table 2.**

| 2θ Values, interspace distances and relative intensities of the diffraction peaks of approximately monohydrate pantoprazole sodium monosolvate with methyl isobutyl ketone, wherein the more intense diffraction peaks are observed at 4.86, 12.90, 16.68, 21.09, 22.77 and 31.65 in 2θ. | | |
|---|---|---|
| Diffraction angles (2θ, °) | Interspace distances (å) | I/Imax (%) |
| 4.860 | 18.1679 | 100 |
| 5.640 | 15.6570 | 7 |
| 9,780 | 9.0365 | 4 |
| 11.010 | 8.0296 | 3 |
| 12.090 | 7.3146 | 4 |
| 12.900 | 6.8571 | 13 |
| 14.820 | 5.9728 | 5 |
| 15.570 | 5.6867 | 7 |
| 16.230 | 5.4569 | 6 |
| 16.680 | 5.3107 | 11 |
| 17.040 | 5.1993 | 7 |
| 17.880 | 4.9569 | 4 |
| 20.520 | 4.3247 | 7 |
| 21.090 | 4.2091 | 10 |
| 21.570 | 4.1165 | 6 |
| 22.050 | 4.0280 | 7 |
| 22.770 | 3.9022 | 20 |
| 23.640 | 3.7605 | 5 |
| 24.330 | 3.6554 | 7 |
| 25.860 | 3.4425 | 7 |
| 26.640 | 3.3435 | 8 |
| 28.380 | 3.1423 | 5 |
| 28.980 | 3.0786 | 4 |
| 29.820 | 2.9938 | 4 |
| 31.650 | 2.8247 | 19 |
| 34.140 | 2.6242 | 5 |
| 34.860 | 2.5716 | 4 |
| 37.770 | 2.3799 | 3 |

**Table 3.**

| 2θ Values, interspace distances and relative intensities of the diffraction peaks of approximately monohydrate pantoprazole sodium monosolvate with diethyl ketone; wherein the more intense diffraction peaks are observed at 5.22, 15.72, 25.05, 31.44 in 2θ. | | |
|---|---|---|
| Diffraction angles (2θ, °) | Interspace distances (å) | I/Imax (%) |
| 5.220 | 16.9157 | 100 |
| 10.380 | 8.5155 | 6 |
| 12.240 | 7.2253 | 4 |
| 13.080 | 6.7631 | 7 |
| 15.030 | 5.8898 | 8 |
| 15.720 | 5.6328 | 9 |
| 17.520 | 5.0579 | 3 |
| 18.150 | 4.8837 | 3 |
| 20.910 | 4.2449 | 3 |
| 22.230 | 3.9958 | 3 |
| 22.830 | 3.8921 | 1 |
| 24.240 | 3.6688 | 4 |
| 25.050 | 3.5520 | 14 |
| 25.650 | 3.4702 | 6 |
| 26.670 | 3.3398 | 4 |
| 27.360 | 3.2571 | 3 |
| 28.290 | 3.1521 | 4 |
| 29.220 | 3.0539 | 2 |
| 29.640 | 3.0115 | 2 |
| 31.440 | 2.8431 | 10 |
| 32.730 | 2.7339 | 2 |
| 34.770 | 2.5781 | 4 |
| 35.460 | 2.5295 | 2 |

**Table 4.**

| 2θ Values, interspace distances and relative intensities of the diffraction peaks of approximately monohydrate pantoprazole sodium monosolvate with acetone, wherein the more intense diffraction peaks are observed at 5.52, 16.32, 26.04 in 2θ. | | |
|---|---|---|
| Diffraction angles (2θ, °) | Interspace distances (å) | I/Imax (%) |
| 5.520 | 15.9971 | 100 |
| 10.350 | 8.5401 | 2 |
| 11.070 | 7.9862 | 1 |
| 11.820 | 7.4811 | 4 |
| 12.840 | 6.8890 | 4 |
| 13.740 | 6.4397 | 6 |
| 15.300 | 5.7864 | 5 |
| 16.320 | 5.4270 | 7 |
| 17.220 | 5.1453 | 3 |
| 18.360 | 4.8283 | 2 |
| 19.470 | 4.5555 | 2 |
| 19.770 | 4.4871 | 2 |
| 20.520 | 4.3247 | 1 |
| 20.760 | 4.2753 | 2 |
| 21.570 | 4.1165 | 4 |
| 22.260 | 3.9904 | 2 |
| 22.920 | 3.8770 | 3 |
| 23.400 | 3.7986 | 2 |
| 24.420 | 3.6422 | 3 |
| 25.230 | 3.5270 | 2 |
| 25.410 | 3.5024 | 3 |
| 26.040 | 3.4191 | 10 |
| 27.810 | 3.2054 | 2 |
| 28.380 | 3.1423 | 2 |
| 29.850 | 2.9908 | 2 |
| 30.060 | 2.9704 | 2 |
| 31.260 | 2.8591 | 1 |
| 32.490 | 2.7536 | 1 |
| 33.360 | 2.6837 | 2 |
| 34.140 | 2.6242 | 1 |
| 35.550 | 2.5233 | 2 |
| 37.620 | 2.3890 | 1 |

### DETAILED DISCLOSURE OF THE INVENTION

A first object of the invention is a novel approximately monohydrate monosolvate crystalline form of pantoprazole sodium which can be represented by the formula 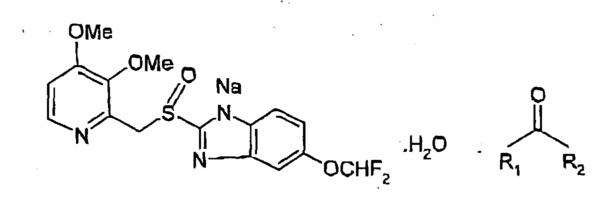 wherein each of R₁ and R₂ is, independently, a straight or branched C₁-C₄ alkyl group optionally substituted with halogen, an aryl-C₁-C₄ alkyl group, an aryl group, a C₄-C₈ alicyclic group, or R₁ and R₂, taken together, form a C₄-C₈ alicyclic ring.

Examples of alkyl groups are methyl, ethyl, n-propyl, i-propyl, n-butyl, i-butyl or t-butyl groups, in particular methyl, ethyl or i-butyl. When an alkyl group is substituted with halogen, it is substituted with one to nine halogen atoms, preferably 1 to 3, independently selected from fluorine, chlorine and bromine. Examples of aryl-alkyl groups are benzyl or phenylethyl groups, preferably benzyl. Examples of aryl groups are phenyl or naphthyl groups, preferably phenyl. Examples of alicyclic groups or alicyclic rings are, respectively, a cyclopentyl or cyclohexyl group or ring.

Preferred examples of the compounds of the invention are those in which, in the ketone of formula R₁(CO)R₂, as defined above, each of R₁ and R₂ is independently straight or branched C₁-C₄ alkyl.

Specific examples of compounds of the invention are:
- pantoprazole sodium in the crystalline form approximately monohydrate monosolvate with acetone;
- pantoprazole sodium in the crystalline form approximately monohydrate monosolvate with methyl ethyl ketone;
- pantoprazole sodium in the crystalline form approximately monohydrate monosolvate with diethyl ketone; and
- pantoprazole sodium in the crystalline form approximately monohydrate monosolvate with methyl isobutyl ketone.

According to present invention, "approximately monohydrate" means a crystalline solid having a water content of approx. 0.8-1.2 moles per mole of pantoprazole sodium, preferably approx. from 0.9 to 1.1 moles. "Approximately monosolvate" means a crystalline solid having a ketone solvent content of approx. 0.8-1.2 moles of solvent per mole of pantoprazole sodium, preferably approx. from 0.9 to 1.1 moles.

The crystalline forms object of the present invention can be prepared by means of a process comprising the formation of a pantoprazole sodium solution in a ketone solvent of formula R₁(CO)R₂, as defined above, in the presence of water, and the subsequent recovery of pantoprazole sodium in the crystalline form approximately monohydrate monosolvate.

Alternatively, this crystalline form can be prepared starting from a solution obtained mixing, in addition to water and the ketone solvent, also an organic co-solvent such as n-hexane, n-heptane, isopropyl ether, ethyl acetate, toluene or an alkanol, such as methanol, ethanol or isopropanol, then recovering pantoprazole sodium in the crystalline form approximately monohydrate monosolvate.

More particularly, pantoprazole sodium in the crystalline form can be recovered from the resulting clear solution by cooling.

Alternatively, the solution can be concentrated by distilling off the ketone solvent, the water and the co-solvent, if present. The product is then recovered by cooling, filtration and drying, preferably under vacuum. The resulting product is dried at a temperature depending on the ketone solvent and any co-solvents used in the process and preferably ranges approx. from 0°C to the boiling temperature of the ketone solvent or ketone solvent/co-solvent mixture used, preferably approx. from 20 to 50°C.

The water percentage compared with the ketone solvent volume used can range approx. from 0.1% to 95% v/v, preferably approx. from 0.2 to 60% v/v.

When present, the organic co-solvent can be added in the desired amount, preferably up to about 5 times v/v the ketone solvent.

The concentration of pantoprazole sodium in the solution can range from 1 to 70% w/w, preferably from 5 to 40%.

Pantoprazole sodium, used as starting material, can be dissolved in the ketone solvent in the presence of water, and, if necessary, the co-solvent.

Alternatively, pantoprazole sodium can be prepared directly *in situ,* for example by dissolving pantoprazol in the acid form in a ketone solvent, in the presence of water and optionally of the co-solvent, and adding a suitable sodium containing basic agent. Examples of such basic agents are sodium hydroxide, sodium carbonate, sodium bicarbonate, sodium alkoxides, e.g. sodium methoxide and sodium t-butoxide, sodium phosphates, such as Na₂HPO₄ and Na₃PO₄.

Alternatively, the crystalline forms of the present invention can be prepared treating a pantoprazole sodium salt aqueous solution with a ketone solvent of formula R₁(CO)R₂, as defined above. The pantoprazole sodium aqueous solution can be prepared by dissolving pantoprazole sodium in water or preparing pantoprazole sodium in situ by treatment of an aqueous suspension of pantoprazol in the acid form with a suitable basic agent containing sodium. Examples of basic agents useful for the purposes of the present invention are those mentioned above. The resulting pantoprazol salt aqueous solution is added with the ketone solvent. The formed solid product is then recovered by filtration and drying, as indicated above.

The resulting novel crystalline forms have purity at least above 99.5%.

The present invention also relates to a process for the purification of pantoprazole sodium salt sesquihydrate or approximately monohydrate pantoprazole sodium salt, comprising the conversion of said forms to a crystalline form approximately monohydrate and monosolvate with a ketone solvent and the reconversion of this to pantoprazole sodium salt sesquihydrate or pantoprazole sodium salt approximately monohydrate, respectively.

According to the invention, pantoprazole sodium salt sesquihydrate or pantoprazole sodium salt approximately monohydrate are used as the starting material for the preparation of a solution, from which the crystalline form approximately monohydrate and monosolvate with a ketone solvent are obtained as described above. A solution of the latter is in turn the starting material for the preparation of pantoprazole sodium salt sesquihydrate or pantoprazole sodium salt approximately monohydrate according to known methods.

Pantoprazole sodium salt sesquihydrate and pantoprazole sodium salt approximately monohydrate are obtained in good yields and high purity level, at least above 99.5%, i.e. such as to fulfill the regulatory requirements, these hydrate forms with high purity level are a further object of the invention.

Pantoprazol in the novel crystalline form approximately monohydrate monosolvate with a ketone solvent can be used in therapy for the treatment of those pathologies in which pantoprazole sodium salt sesquihydrate is used, substantially at the same dosage.

A further object of the invention is to provide a compound of formula for use as a medicament, in particular as agent inhibiting gastric acid secretion.

The invention also relates to a pharmaceutical composition comprising a compound of formula, optionally at least one of pantoprazole sodium salt sesquihydrate and pantoprazole sodium salt approximately monohydrate, as the active ingredient, together with a pharmaceutically acceptable excipient and/or carrier.

A pharmaceutical composition of the invention can be formulated according to known methods in any pharmaceutical form known in the art for the administration to mammals, including humans.

One of the advantages of the novel crystalline form of the invention is its use in the pharmaceutical technique, in particular in connection with procedures such as filtration, drying, sieving, formulation, storage, and the like.

The following examples further illustrate the invention.

### EXAMPLE 1

### Preparation of pantoprazole sodium salt approximately monohydrate monosolvate with methyl isobutyl ketone

5 g of 5-(difluoromethoxy)-2-[[(3,4-dimethoxy-2-pyridinyl)methyl] sulfinyl]-1H-benzimidazole sodium salt monohydrate (11.8 mmoles) are dissolved in 15 ml of purified water, under stirring. 20 ml of methyl isobutyl ketone are slowly dropped into resulting solution, stirring vigorously the mixture at room temperature. One hour after completion of the addition, a precipitate forms at the interphase which is recovered by filtration with suction, washed on the filter with methyl isobutyl ketone and dried under vacuum at a temperature of 50°C to constant weight. 4.3 g of product are obtained (molar yield: 69.6%).

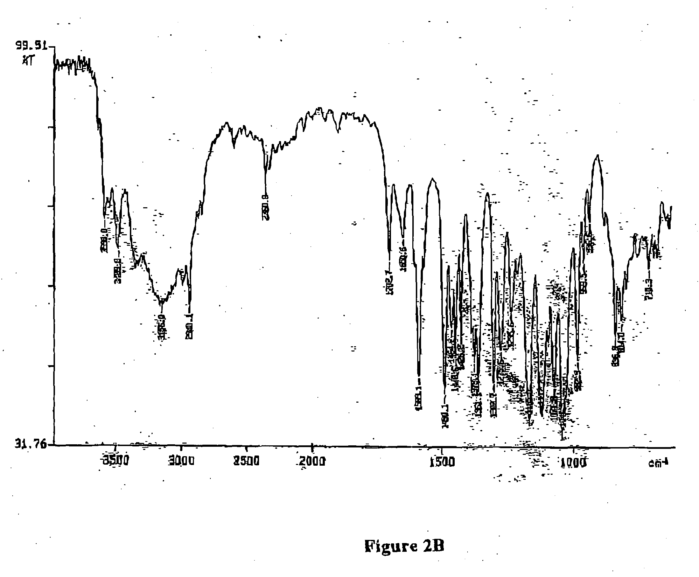

XRPD analysis shows that the solid is a substantially crystalline product, having the following characteristics: crystallinity as shown by X-ray powder diffraction pattern of Figure 2A, characterized by a multiplicity of diffraction peaks, the more intense being at 4.86, 12.90, 16.68, 21.09, 22.77, 31.65 in 2θ as shown in Table 2, IR Spectrum of Figure 2B, 3.5% w/w water content (measured by Karl Fischer) and 18.8% w/w methyl isobutyl ketone content (measured by ¹H-NMR).

### EXAMPLE 2

### Preparation of pantoprazole sodium salt approximately monohydrate monosolvate with methyl ethyl ketone

55 kg of pantoprazole sodium salt sesquihydrate (127.3 moles) are dissolved at about 75°C in 265 kg of methyl ethyl ketone. The resulting solution is slowly added with water (4.5 kg). The resulting mixture is cooled to 42-45°C and kept under these conditions until a solid product forms. The mixture is then cooled to 15-20°C and kept under these conditions for at least an hour. The formed solid is filtered and washed with methyl ethyl ketone, then dried under vacuum at 40°C to constant weight. 53.5 kg product are obtained (molar yield 84.9%).

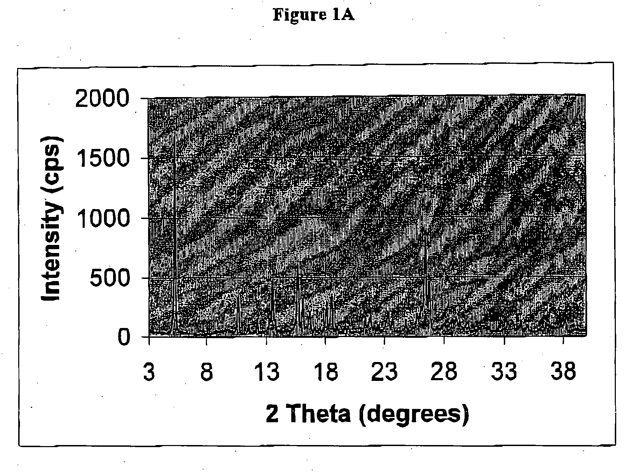

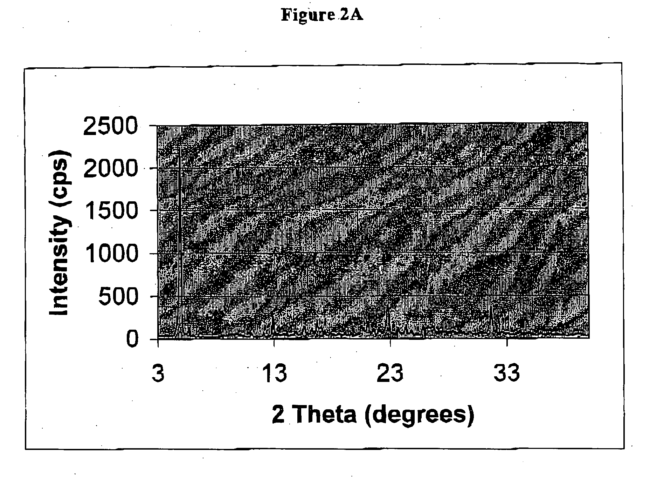

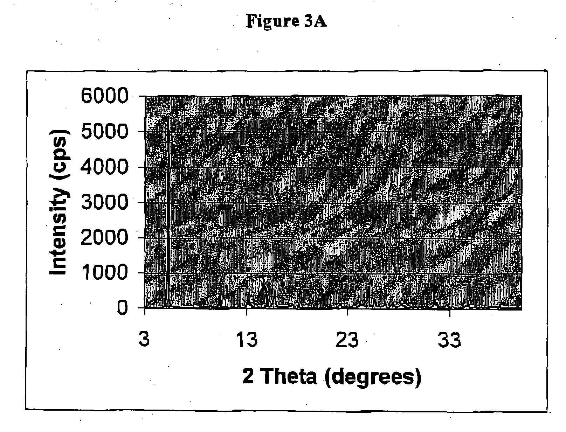

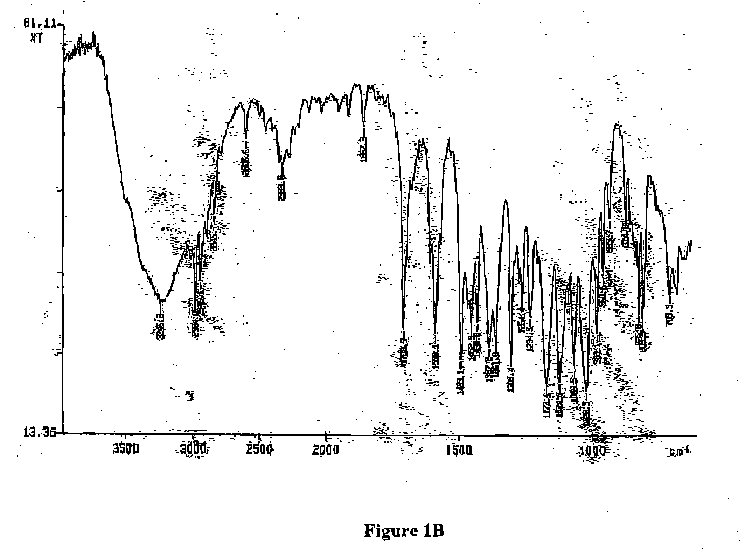

XRPD analysis shows that the solid is a substantially crystalline product, having the following characteristics: crystallinity as shown by X-ray powder diffraction pattern of Figure 1A, characterized by a multiplicity of diffraction peaks, the more intense being at 5.31, 13.53, 15.66, 16.08, 18.51, 26.43, 26.76 in 2θ as shown in Table 1, IR Spectrum of Figure 1B, 3.6% w/w water content (measured by Karl Fischer) and 14.6% w/w methyl isobutyl ketone content (measured by ¹H-NMR).

### EXAMPLE 3

### Preparation of pantoprazole sodium salt approximately monohydrate monosolvate with acetone

31.2 g of pantvprazol in the acid form (81.5 mmoles) are suspended in 210 ml of acetone. Then 6.5 g of a 50% w/w NaOH aqueous solution (81.5 mmoles) are slowly added. The resulting mixture is refluxed to complete dissolution of the solid, then cooled to 45°C. A precipitate forms, which is cooled to 15°C and kept under these conditions for an hour. The solid is filtered, washed with some acetone and dried at about 30°C under vacuum to constant weight. 31.5 g of product are obtained (molar yield 80.4%).

XRPD analysis shows that the solid is a substantially crystalline product, having the following characteristics: crystallinity as shown by X-ray powder diffraction pattern characterized by a multiplicity of diffraction peaks, the more intense being at 5.52, 16.32, 26.04 in 2θ as shown in Table 4. The solid has 3.8% w/w water content (measured by Karl Fischer) and 12% w/w methyl isobutyl ketone content (measured by ¹H-NMR).

### EXAMPLE 4

### Preparation of pantoprazole sodium salt approximately monohydrate monosolvate with diethyl ketone

5 g of 5-(difluoromethoxy)-2-[[(3,4-dimethoxy-2-pyridinyl)methyl] sulfinyl]-1H-benzimidazole sodium salt monohydrate (11.8 mmoles) are dissolved in 15 ml of purified water, under stirring. 20 ml of diethyl ketone are slowly dropped into the resulting solution, stirring vigorously the mixture at room temperature. One hour after completion of the addition, a precipitate forms at the interphase which is recovered by filtration under suction, washed on the filter with diethyl ketone and dried under vacuum at a temperature of 50°C to constant weight, 4.5 g of product are obtained (molar yield: 74.9%).

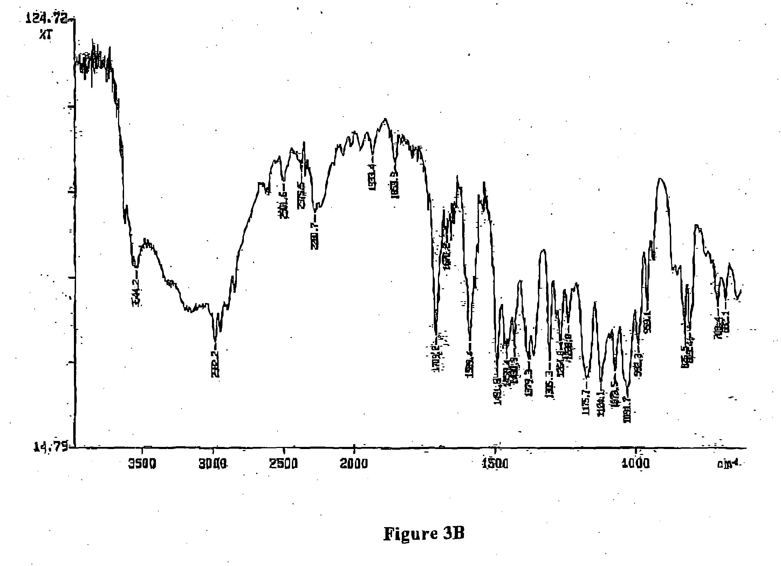

XRPD analysis shows that the solid is a substantially crystalline product, having the following characteristics: crystallinity as shown by X-ray powder diffraction pattern of Figure 3A, characterized by a multiplicity of diffraction peaks, the more intense being at 5.22, 15.72, 25.05, 31.44 in 2θ as shown in Table 3, IR Spectrum of Figure 3B, 3.6% w/w water content (measured by Karl Fischer) and 16.8% w/w diethyl ketone content (measured by ¹H-NMR).

## Claims

1. Crystalline form of approximately monohydrate and monosolvate pantoprazole sodium which can be represented by the formula 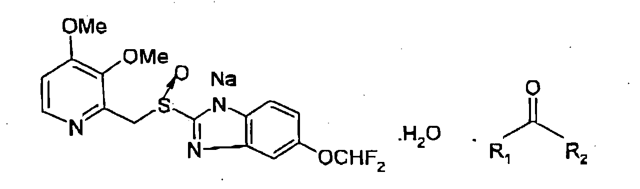 wherein each of R₁ and R₂, independently, is a straight or branched C₁-C₄ alkyl group optionally substituted with halogen, an aryl-C₁-C₄ alkyl group, an aryl group, a C₄-C₈ alicyclic group, or R₁ and R₂, taken together, form a C₄-C₈ alicyclic ring.

2. Crystalline form according to claim 1, having a water content of approx. 0.8-1.2 moles per mole of pantoprazole sodium and a content in ketone solvent of approx. 0.8-1.2 moles per mole of pantoprazole sodium.

3. Crystalline form according to claim 2, having a water content of approx. 0.9 to 1.1 moles per mole of pantoprazole sodium and a ketone solvent content of approx. 0.9 to 1.1 moles per mole of pantoprazole sodium.

4. Crystalline form according to any one of claims 1 to 3, wherein each of R₁ and R₂ is independently straight or branched C₁-C₄ alkyl.

5. Crystalline form according to any one of claims 1 to 4, which is:
• pantoprazole sodium in the crystalline form approximately monohydrate monosolvate with acetone;
• pantoprazole sodium in the crystalline form approximately monohydrate monosolvate with methyl ethyl ketone;
• pantoprazole sodium in the crystalline form approximately monohydrate monosolvate with diethyl ketone; and
• pantoprazole sodium in the crystalline form approximately monohydrate monosolvate with methyl isobutyl ketone.

6. Crystalline form of pantoprazole sodium in form approximately monohydrate monosolvate with acetone according to claim 5, having the more intense diffraction peaks at 5.52, 16.32, 26.04 in 2θ.

7. Crystalline form of pantoprazole sodium in the crystalline form approximately monohydrate monosolvate with methyl ethyl ketone according to claim 5, having more intense diffraction peaks at 5.31, 13.53, 15.66, 16.08, 18.51, 26.43 and 26.76 in 2θ.

8. Crystalline form of pantoprazole sodium in the crystalline form approximately monohydrate monosolvate with diethyl ketone according to claim 5, having the more intense diffraction peaks a 5.22, 15.72, 25.05, 31.44 in 2θ.

9. Crystalline form of pantoprazole sodium in the crystalline form approximately monohydrate monosolvate with methyl isobutyl ketone according to claim 5, having the more intense diffraction peaks at 4.86, 12.90, 16.68, 21.09, 22.77 and 31.65 in 2θ.

10. Pharmaceutical composition comprising a compound of formula (I), as defined in claim 1, optionally at least one of pantoprazole sodium salt sesquihydrate and pantoprazole sodium salt approximately monohydrate, as active ingredient, and a pharmaceutically acceptable excipient and/or carrier.

11. A process for the purification of pantoprazole sodium salt sesquihydrate or pantoprazole sodium salt approximately monohydrate, comprising the conversion of said forms to a crystalline form approximately monohydrate and monosolvate with a ketone solvent, as defined in claim 1, and its reconversion to pantoprazole sodium salt sesquihydrate or pantoprazole sodium salt approximately monohydrate, respectively.

12. Pantoprazole sodium salt sesquihydrate and pantoprazole sodium salt approximately monohydrate, having a purity degree at least above 99.5%.
